# EUROPÄISCHE PATENTSCHRIFT

(11) **EP 1 395 331 B1**
(45) Veröffentlichungstag und Bekanntmachung des Hinweises auf die Patenterteilung: **01.06.2005**
(21) Anmeldenummer: 02732367.4
(22) Anmeldetag: 30.03.2002
(51) Int. Cl.: A61N 1/20, A61N 1/32

(54) **VORRICHTUNG ZUR BEHANDLUNG VON TUMOREN**
DEVICE FOR THE TREATMENT OF TUMOURS
DISPOSITIF POUR TRAITER DES TUMEURS

(30) Priorität: 30.05.2001 DE 20109099 U
(43) Veröffentlichungstag der Anmeldung: 10.03.2004
(73) Patentinhaber: Schönfeld, Andreas, 76187 Karlsruhe (DE)
(72) Erfinder: Schönfeld, Andreas, 76187 Karlsruhe (DE)
(74) Vertreter: Dimmerling, Heinz, Dipl.-Ing.
(86) Internationale Anmeldenummer: PCT/DE2002/001178
(87) Internationale Veröffentlichungsnummer: WO 2002/096511

(56) Entgegenhaltungen:
- DE-A- 4 344 986
- FR-A- 2 694 501
- GB-A- 2 281 863
- US-A- 5 873 849

## Beschreibung

Die Erfindung betrifft eine Vorrichtung zur Behandlung von Tumoren nach dem Oberbegriff des Anspruchs 1, mit wenigstens einem eine Stromquelle aufweisenden Generator, und wenigstens zwei nadelförmigen Elektroden, mittels welchen Strom in den Tumor ein- beziehungsweise aus dem Tumor ausleitbar ist.

Eine derartige Vorrichtung ist beispielsweise aus der Druckschrift "Percutaneous bio-electrotherapy of ' cancerous tumours", Dr. med. Rudolf Pekar, Verlag Wilhelm Maudrich, Wien-München-Bem, 1997, bekannt. Die bekannte Vorrichtung weist zwei nadelförmige Elektroden auf; eine zum Einleiten von Strom in den Tumor, eine zur Ausleitung von Strom aus dem Tumor. Mittels der bekannten Vorrichtung wird Gleichstrom durch einen Tumor geleitet. Dies geschieht derart, daß die zwei nadelförmigen Elektroden in den Tumor eingestochen werden und anschließend an die Elektroden eine Gleichspannung angelegt wird, so daß durch den Tumor ein Gleichstrom fließt. Hierdurch sollen Krebszellen abgetötet werden.

Es hat sich gezeigt, daß das Verfahren um so wirksamer ist, je mehr Strom durch den Tumor geleitet wird. Man ist daher bestrebt, den Strom so groß wie möglich zu wählen. Da es sich aber gezeigt hat, daß bei einem Strom ab etwa 80 Milliampere das Gewebe um die Einstichstelle der nadelförmigen Elektrode herum in unerwünschter Weise beschädigt wird, läßt sich der Strom bei einer Vorrichtung mit nadelförmigen Elektroden nicht beliebig erhöhen.

Die aus der genannten Druckschrift bekannte Vorrichtung weist zwei Systeme mit separaten Ausgängen auf. Es wäre somit denkbar, beide Systeme parallel zur Behandlung desselben Tumors zu verwenden. Hierdurch könnte zwar der in den Tumor eingeleitete Strom verdoppelt werden, wobei der jeweils mittels einer Elektrode in den Tumor eingeleitete Strom auf einen Maximalwert begrenzt wird. Da sich der Strom seinen Weg durch den Tumor aber selbst sucht, ist nicht gewährleistet, daß der Strom entsprechend seiner Einleitung über die jeweils zugehörige Elektrode wieder ausgeleitet wird. Es besteht vielmehr die Gefahr, daß aufgrund der inhomogenen elektrischen Leitfähigkeit des Tumors der Strom sich einen solchen Weg durch den Tumor sucht, daß er überwiegend über eine Elektrode wieder austritt und über die andere Elektrode lediglich ein Reststrom aus dem Tumor ausgeleitet wird. Hierdurch würde das Gewebe um die nadelförmige Elektrode herum, über welche der größte Teil des Stromes aus dem Tumor austritt, zerstört werden. Es ist daher in der Druckschrift auch nicht angegeben, daß die beiden Systeme parallel zur Behandlung eines Tumors verwendet werden können, sondem angegeben, daß die beiden Systeme zur Behandlung von zwei Patienten vorgesehen sind.

Es ist Aufgabe der Erfindung, eine eingangs genannte Vorrichtung derart auszubilden, daß sie zur problemlosen Behandlung von Tumoren mit höherem Strom geeignet ist.

Die Lösung dieser Aufgabe ergibt sich aus den Merkmalen des kennzeichnenden Teils des Anspruchs 1. Vorteilhafte Weiterbildungen der Erfindung ergeben sich aus den Unteransprüchen.

Gemäß der Erfindung ist eine Vorrichtung zur Behandlung von Tumoren, mit wenigstens einem eine Stromquelle aufweisenden Generator, und wenigstens zwei nadelförmigen Elektroden mittels welchen Strom in den Tumor einbeziehungsweise aus dem Tumor ausleitbar ist, dadurch gekennzeichnet, daß eine Steuerung vorhanden ist, mittels welcher der Strom durch jede Elektrode jeweils auf einen einstellbaren Maximalwert begrenzbar ist.

Dadurch, daß eine Steuerung vorhanden ist, mittels welcher der Strom durch die Elektroden jeweils auf einen Maximalwert begrenzbar ist, wird in vorteilhafter Weise erreicht, daß an keiner Elektrode mehr Strom in den Tumor eingeleitet beziehungsweise aus dem Tumor ausgeleitet wird, als ohne Beschädigung des Gewebes möglich ist. Durch die Steuerung lassen sich somit mehrere Elektroden gleichzeitig verwenden.

Denn, da der maximale Strom durch die Elektroden auf einen Maximalwert begrenzt ist, bleibt es ohne Auswirkungen, wenn beispielsweise eine Elektrode an einer Stelle in den Tumor eingestochen ist, welche für mehrere von unterschiedlichen Elektroden ausgehenden durch den Tumor fließenden elektrischen Strömen wegen einer hohen elektrischen Leitfähigkeit als bevorzugter Weg aus dem Tumor heraus gewählt werden würde. Durch die Begrenzung des Stroms durch die Elektrode auf einen Maximalwert, sind die Ströme gezwungen, sich durch den Tumor andere Wege zu anderen Elektroden zu suchen.

Als besonders vorteilhaft hat sich eine Ausführungsform der Erfindung herausgestellt, bei der jede Elektrode von einer separaten Stromquelle gespeist wird. Das heißt, daß jede Elektrode, mittels welcher Strom in den Tumor eingeleitet wird, mit einer separaten Stromquelle verbunden ist. Ebenso ist jede Elektrode, mittels welcher Strom aus dem Tumor ausgeleitet wird, mit einer Stromquelle, oder besser ausgedrückt, mit einer Stromsenke verbunden, wobei die Richtung des Stroms zu den erstgenannten Stromquellen umgekehrt ist. Durch die Versorgung der Elektroden mittels Stromquellen beziehungsweise Stromsenken läßt sich die Begrenzung des durch die Elektroden fließenden Stroms auf einen Maximalwert auf einfache Weise realisieren.

Besonders vorteilhaft ist es, wenn die Stromquellen als Konstantstromquellen ausgebildet sind.

Bei einer weiteren besonderen Ausführungsform der Erfindung ist vorgesehen, daß der von den Stromquellen abgegebene Strom wenigstens bei den Stromquellen einstellbar ist, mittels welcher Strom in den Tumor einleitbar ist. Hierdurch ist es auf einfache Weise möglich, den Stromfluß durch den Tumor lokal unterschiedlich vorzunehmen. Der Stromfluß durch den Tumor kann weiter noch dadurch beeinflußt werden, daß die von den Stromsenken aufgenommenen Ströme ebenfalls einstellbar sind. Unbeschadet der Einstellbarkeit der von den Stromquellen abgegebenen beziehungsweise von den Stromsenken aufgenommenen Strömen bleibt die Begrenzung der Ströme auf einen jeweiligen Maximalwert, wobei dieser Maximalwert für alle Stromquellen beziehungsweise Stromsenken gleich sein kann.

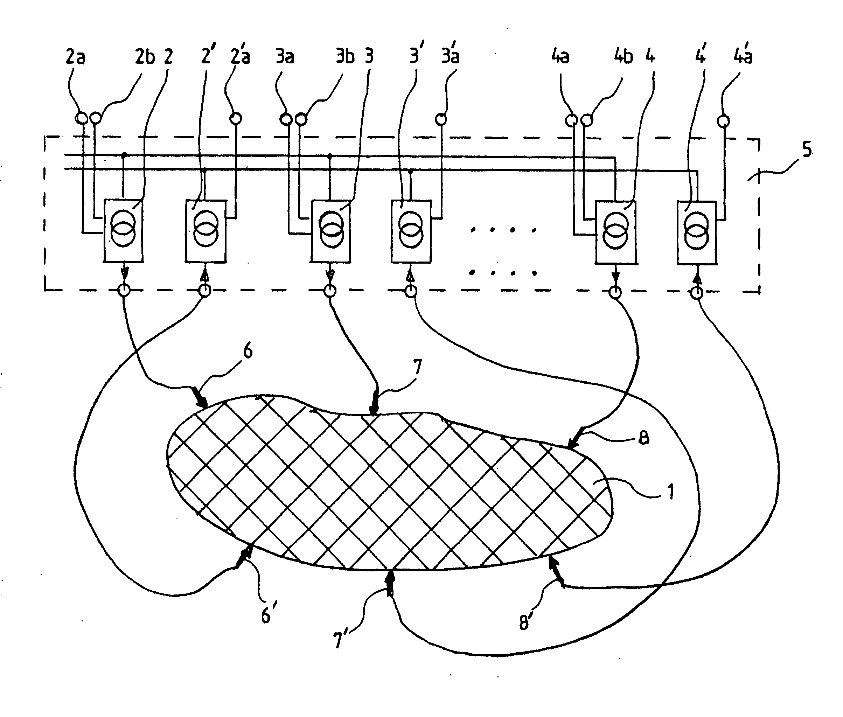

Weitere Einzelheiten, Merkmale und Vorteile der vorliegenden Erfindung ergeben sich aus der nachfolgenden Beschreibung eines besonderen Ausführungsbeispiels unter Bezugnahme auf die Zeichnung.

Es zeigt die einzige Figur eine Ausführungsform einer erfindungsgemäßen Vorrichtung.

Wie der Figur entnommen werden kann, weist ein Generator 5 mehrere Stromquellen 2, 3, 4 sowie mehrere zugehörige Stromsenken 2', 3', 4' auf. Die Ausgänge der Stromquellen 2, 3, 4 sind mit nadelförmigen Elektroden 6, 7, 8 verbunden. Die Ausgänge der Stromsenken 2', 3', 4' sind mit nadelförmigen Elektroden 6', 7'. 8' verbunden. Die nadelförmigen Elektroden 6, 6', 7, 7', 8, 8' sind in das Zellgewebe eines Tumors 1 eingestochen.

Die Stromquellen 2, 3, 4 weisen jeweils einen Steuereingang 2a, 3a, 4a auf, mittels welchem der Maximalwert des von der jeweiligen Stromquelle abgegebenen Stromes einstellbar ist. Des weiteren weisen die Stromquellen 2, 3, 4 jeweils einen weiteren Steuereingang 2b, 3b, 4b auf, mittels welchem der von der jeweiligen Stromquelle abgegebene Strom einstellbar ist. Die Stromsenken 2', 3', 4' weisen ähnlich wie die Stromquellen 2, 3, 4 einen Steuereingang 2'a, 3'a, 4'a auf, mittels welchem jeweils der Maximalwert des von der Stromsenke aufgenommenen Stromes einstellbar ist.

Sind die Stromquellen 2, 3, 4 so eingestellt, daß sie jeweils einen Strom von beispielsweise 80 Milliampere abgeben, und sind die Stromsenken 2', 3', 4' so eingestellt, daß der maximal von in den jeweils aufgenommenen Strom beispielsweise auf 80 Milliampere begrenzt ist, läßt sich der Tumor 1 mit 240 Milliampere behandeln, ohne daß die Gefahr besteht, daß das Gewebe an der Einstichstelle einer Elektrode beschädigt wird. Denn beispielsweise selbst wenn die elektrische Leitfähigkeit des Tumors 1 so ausgebildet wäre, daß ein Teil des Stromes, der mittels der Elektrode 7 in den Tumor 1 eingeleitet wird, zur Elektrode 6' hinfließen würde, und der von der Elektrode 6 in den Tumor 1 eingeleitete Strom vollständig zur Elektrode 6' fließen würde, so würde durch die Begrenzung des Maximalstroms der Stromsenke 2' auf 80 Millimampere verhindert werden, daß der betreffende Teil des mittels der Elektrode 7 in den Tumor 1 eingeleiteten Stromes über die Elektrode 6' aus dem Tumor 1 ausgeleitet wird. Der Strom muß sich daher einen anderen Weg zur Elektrode 7' suchen.

Es versteht sich von selbst, daß, obwohl in der Figur jeweils nur drei Stromquellen und drei Stromsenken dargestellt sind, eine viel größere Anzahl von Stromquellen und Stromsenken verwendet werden kann. Dies wird auch bereits durch die in der Figur enthaltenen gepunkteten Linien angedeutet.

## Patentansprüche

1. Vorrichtung zur Behandlung von Tumoren (1), mit wenigstens einem eine Stromquelle (2, 2', 3, 3', 4, 4') aufweisenden Generator (5), und wenigstens zwei nadetförmigen Elektroden (6, 6', 7, 7', 8, 8') mittels welchen Strom in den Tumor (1) ein- beziehungsweise aus dem Tumor (1) ausleitbar ist,
**dadurch gekennzeichnet,**
**daß** eine Steuerung vorhanden ist, mittels welcher der Strom durch jede Elektrode (6, 6', 7, 7', 8, 8') jeweils auf einen einstellbaren Maximalwert begrenzbar ist.

2. Vorrichtung nach Anspruch 1,
**dadurch gekennzeichnet,**
**daß** jede Elektrode (6, 6', 7, 7', 8, 8') von einer separaten Stromquelle (2, 2', 3, 3', 4, 4') gespeist wird.

3. Vorrichtung nach Anspruch 1 oder 2,
**dadurch gekennzeichnet,**
**daß** die Stromquellen (2, 2', 3, 3', 4, 4') als Konstantstromquellen ausgebildet sind.

4. Vorrichtung nach einem der Ansprüche 1 bis 3,
**dadurch gekennzeichnet,**
**daß** der von den Stromquellen (2, 2', 3, 3', 4, 4') abgegebene Strom wenigstens bei den Stromquellen (2, 3, 4) einstellbar ist, mittels welcher Strom in den Tumor (1) einleitbar ist.

## Claims

1. Device for the treatment of tumours (1), having at least one generator (5) with a current source (2, 2', 3, 3', 4, 4') and at least two needle-shaped electrodes (6, 6', 7, 7', 8, 8') by means of which current may be fed into or out of the tumour (1), **characterised in that** a control system is present by means of which the current through each electrode (6, 6', 7, 7', 8, 8') may be limited to an adjustable maximum value.

2. Device according to claim 1, **characterised in that** each electrode (6, 6', 7, 7', 8, 8') is fed by a separate current source (2, 2', 3, 3', 4, 4').

3. Device according to claim 1 or 2, **characterised in that** the current sources (2, 2', 3, 3', 4, 4') are designed as constant current sources.

4. Device according to one of the claims 1 to 3, **characterised in that** the current supplied by the current sources (2, 2', 3, 3', 4, 4') is adjustable at least for the current sources (2, 3, 4), by means of which current may be fed into the tumour (1).

## Revendications

1. Dispositif pour traiter des tumeurs (1) comprenant au moins un générateur (5) présentant une source de courant (2, 2', 3, 3', 4, 4') et au moins deux électrodes en forme d'aiguille (6, 6', 7, 7', 8, 8') au moyen desquelles on peut faire entrer du courant dans la tumeur (1), respectivement faire sortir du courant de la tumeur (1),
**caractérisé par le fait**
**qu'**il est prévu une commande au moyen de laquelle on peut limiter le courant à travers chaque électrode (6, 6', 7, 7', 8, 8') à une valeur maximale réglable.

2. Dispositif selon la revendication 1,
**caractérisé par le fait**
**que** chaque électrode (6, 6', 7, 7', 8, 8') est alimentée par une source de courant (2, 2', 3, 3', 4, 4') séparée.

3. Dispositif selon la revendication 1 ou 2,
**caractérisé par le fait**
**que** les sources de courant (2, 2', 3, 3', 4, 4') sont formées de sources de courant constant.

4. Dispositif selon l'une des revendications 1 à 3,
**caractérisé par le fait**
**que** le courant délivré par les sources de courant (2, 2', 3, 3', 4, 4') est réglable au moins sur les sources de courant (2, 3, 4) au moyen desquelles on peut faire entrer du courant dans la tumeur (1).
